# EUROPEAN PATENT SPECIFICATION

(11) **EP 3 019 142 B1**
(45) Date of publication and mention of the grant of the patent: **04.10.2017**
(21) Application number: 14736372.5
(22) Date of filing: 03.07.2014
(51) Int. Cl.: A61K 8/25, A61K 8/49, A61Q 17/04, A61K 8/81, A61K 8/891, A61K 8/02

(54) **UV SCREENING COMPOSITION COMPRISING A UV FILTER, AN ORGANOPOLYSILOXANE FUNCTIONALIZED WITH A UV ABSORBER AND POROUS SILICA AND / OR POLYMETHYLMETHACRYLATE PARTICLES**
TOPISCHE UV-SCHUTZUSAMMENSETZUNG ENTHALTEND EINEN UV-FILTER, EIN UV-ABSORBERFUNKTIONALISIERTE-ORGANOPOLYSILOXAN, UND PORÖSE SILICIUMDIOXID UND / ODER POLYMETHYLMETHACRYLAT-TEILCHEN
CRÈME SOLAIRE COMPRENANT UN FILTRE UV, UN ORGANOPOLYSILOXANE FONCTIONNALISÉ PAR UN ABSORBEUR DE RAYONS UV ET DE PARTICULES POREUSE DE LA SILICE ET / OU DE POLYMÉTHACRYLATE DE MÉTHYLE

(30) Priority: 08.07.2013 EP 13175577
(43) Date of publication of application: 18.05.2016
(73) Proprietor: DSM IP Assets B.V., 6411 TE Heerlen (NL)
(72) Inventor: BROCK, Achim, CH-4002 Basel (CH); GSTOETTMAYR, Christian, CH-4002 Basel (CH); HUEBER, Aline, CH-4002 Basel (CH); MENDROK-EDINGER, Christine, CH-4002 Basel (CH); MESAROS, Szilvia, CH-4002 Basel (CH); RADOMSKY, Karina, CH-4002 Basel (CH); SAECKER, Christine, CH-4002 Basel (CH); VOLLHARDT, Juergen, H., CH-4002 Basel (CH)
(74) Representative: Berg, Katja
(86) International application number: PCT/EP2014/064156
(87) International publication number: WO 2015/003987

(56) References cited:
- EP-A1- 0 893 119
- EP-A1- 1 407 757
- WO-A1-2005/107695
- WO-A2-2005/023535
- WO-A2-2012/095786
- GB-A- 2 409 203

## Description

The present invention relates to a topical composition comprising a hydrophilic benzimidazole type UVB filter, an organopolysiloxane functionalized with at least one UV-light absorbing group, and highly porous silica and/ or highly porous cross-linked polymethylmethacrylate beads having a particle size Dᵥ0 of greater 0.3 µm, a Dᵥ100 of less than 35 µm, a Dᵥ50 selected in the range of 9 to 15 µm and an oil absorption capacity selected in the range of 1.2-2.5 cc/g. Furthermore the invention relates to the use of such beads to ameliorate the short and long-term sensory properties of a topical composition comprising a hydrophilic benzimidazole type UVB filter and an organopolysiloxane functionalized with at least one UV-light absorbing group.

Sun care compositions comprising hydrophilic benzimidazole type UVB filter(s) and organopolysiloxane(s) functionalized with at least one UV-light absorbing group for protection of the skin against the adverse effects of UV radiation are well known to a person skilled in the art.

However, due to the increasing demand for sun care products exhibiting a high SPF (Sun Protection Factor), more and more UV-filter substances at elevated levels have to be incorporated into the sun care products; this, however, is not always feasible, as high UV-filter substance concentrations add considerable cost to the formulation and often lead to an unpleasant skin feel and/ or aesthetic appearance. Furthermore, high sunscreen levels can promote increased irritancy.

Thus, there is an ongoing need for substances which are able to boost the SPF and contribute to a good skin feel such as in particular an improved silicone, i.e. velvety, silky, and powdery skin feel. Patent document WO 2012/095786 concerns the provision of UV screening topical products that comprise particulates. Surprisingly it has been found that the addition of specific highly porous silica or cross-linked polymethylmethacrylate beads to a topical composition comprising a hydrophilic benzimidazole type UVB filter and an organopolysiloxane functionalized with at least one UV-light absorbing group significantly enhanced the in vivo SPF. Furthermore, the topical compositions exhibited an improved silicone skin feel.

Thus, the present invention relates to a topical composition comprising a hydrophilic benzimidazole type UVB filter, an organopolysiloxane functionalized with at least one UV-light absorbing group, and highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g.

Another subject matter of the invention is directed to a method enhancing the in vivo SPF of a topical composition comprising a hydrophilic benzimidazole type UVB filter and an organopolysiloxane functionalized with at least one UV-light absorbing group, said method comprising the step of adding to the topical composition highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g and appreciating the effect.

In a further embodiment the invention relates to the use of highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g to enhance the in vivo SPF of a topical composition comprising a hydrophilic benzimidazole type UVB filter and an organopolysiloxane functionalized with at least one UV-light absorbing group.

In another embodiment the invention relates to the use of highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g to enhance the silicone skin feel of a topical composition comprising an organopolysiloxane functionalized with at least one UV-light absorbing group.

In all embodiments of the present invention, the amount of beads is independently of each other preferably selected in the range of 0.5 to 10 wt.-%, more preferably in the range of 1 to 5 wt.-%, and most preferably in the range of 2 to 4 wt.-% based on the total weight of the composition.

It is furthermore preferred if the composition comprises only one kind of beads i.e. either highly porous silica beads or highly porous cross-linked polymethylmethacrylate beads.

The porous silica beads used according to the present invention may be prepared from sodium silicate by emulsion polymerization according to standard procedures such as e.g. via the sol-gel method. They silica beads may be used as such or may be further coated with a suitable coating agent such as e.g. a silicone oil. Suitable silicone oils include in particular non-volatile silicone oils such as dialkyl and alkyl aryl siloxanes as well as alkoxylated and / or aminated derivatives thereof, dihydroxypolydimethylsiloxanes and polyphenylalkylsiloxanes. Preferred silicone oils encompass dimethicone, dimethiconol, dimethicone copolyol, phenyl trimethicone, methicone, simethicone as well as mixtures thereof. Most preferably the silica beads are either uncoated or coated with dimethicone.

The particle size of the beads according to the invention (in volume %) is determined by a Coulter LS13320 or Malvern Mastersizer 2000 according to standard methods in the art. In number % the average particle size Dₙ50 ranges from 9 to 15 µm.

The oil absorption capacity (g/cc) refers to the amount of paraffin (in cc) absorbed by a specified amount (g) of the beads, i.e. the amount until the loose and dry powder disappears. It includes the oil absorption capacity of the dry particles existing between the inherent voids within and on the surface of the particles. The oil absorption capacity as referred to in the present invention is determined at 23°C by weighting 2g of the respective beads into a 20 ml beaker glass. Then liquid paraffin (Paraffinum Perliquidum PH.EUR. CAS 8042-47-5) is added. After addition of 4 to 5 drops of paraffin to the powder, mixing is performed using a spatula, and addition of paraffin is continued until conglomerates of oil and powder have formed. From this point, the paraffin is added one drop at a time and the mixture is then triturated with the spatula. The addition of oil is stopped when the loose and dry powder completely disappears and a highly viscous white to transparent homogeneous gel is obtained. The oil absorption capacity (cc/g) is then calculated by the volume of paraffin used (in cc) per g of the respective beads.

In all embodiments of the present invention the oil absorption capacity of the silica beads is preferably selected in the range of 1.2 to 2.0 cc/g, more preferably in the range of 1.3 to 1.8 cc/g.

In all embodiments of the present invention preferably the oil absorption capacity of the cross-linked polymethylmethacrylate beads selected in the range of 1.5-2.0 cc/g.

Suitable porous silica beads according to the present invention are e.g. obtainable as VALVANCE™ *Touch* 210, respectively VALVANCE™ *Touch* 250 at DSM Nutritional Products Ltd Kaiseraugst.

The highly porous cross-linked polymethylmethacrylate beads according to the present invention are preferably obtained by copolymerization of a monomer mixture consisting of methyl methacrylate and of ethylene glycol dimethacrylate in the presence of a porogen according to known methods in the art and as e.g. outlined in KR 2006036614.

The term 'consisting of' as used according to the present invention means that the total amount of monomers ideally sum up to 100 wt.-%. It is however not excluded that small amount of impurities or additives may be present such as e.g. in amounts of less than 5 wt.-%, preferably less than 3 wt.-% which are e.g. introduced via the respective raw materials.

The porogen is preferably selected from the group consisting of toluene, n-hexanone, methylisobutyl ketone and isoamyl alcohol.

Initiators for polymerizing the monomers to provide the highly porous cross-linked polymethylmethacrylate beads of the invention are those which are normally suitable for free-radical polymerization of acrylate monomers and which are oil-soluble and have low solubility in water such as e.g. organic peroxides, organic peroxyesters and organic azo initiators. The initiator is generally used in an amount of about 0.01 to 1 wt.-% based on the total monomer content.

Optionally, a water soluble inhibitor can be added to inhibit polymerization in the water phase in order to prevent the formation of too much polymer by emulsion and/or solution polymerization in the water phase, which can result in bead agglomeration or emulsion type polymerization. Suitable inhibitors include those selected from thiosulfates, thiocyanates, water soluble hydroquinones and nitrites. When used, the water soluble inhibitor can generally be added in an amount of from about 0.01 to about 1 parts by weight based on 100 parts total monomer content.

Furthermore, a water soluble or water dispersible polymeric stabilizer is needed to stabilize the suspension and in order to obtain stable beads. The stabilizer is preferably a water soluble or water dispersible polymer such as e.g. polyvinylpyrrolidone, polyvinylmethylether, polyethyleneimine, polyvinylalcohol, gelatin, starch, (m)ethylcellulose, carboxymethylcellulose, hydroxyethylcellulose, hydroxypropyl ellulose, poly(meth)acrylic acid and their sodium salts, and the like. The stabilizer is preferably used in an amount of about 0.001 to 10 wt.-%, more preferable in an amount of about 0.01 to 1 wt-% based on the total monomer content.

The monomers, free-radical initiator, and any optional materials can be mixed together in the prescribed ratio to form a premix. The stabilizer can be combined with water and then with the premix to form an oil in water suspension. The resulting suspension typically comprises from about 10 to about 50 weight percent monomer premix and from about 90 to about 50 weight percent water phase. Bead-type suspension polymerization in accordance with the present invention is typically a thermally initiated polymerization and is preferably carried out with agitation for about 2 to about 16 hours at a temperature between about 40° C and 90° C. After isolation of the beads according to standard methods such as filtration or centrifugation the beads are preferably washed e.g. with water and/ or ethanol and subjected to an extended drying, preferably at about 40-100°C and more preferably at about 80-100°C in order to further reduce the residual monomer content to an amount of below 250 ppm such as in particular below 100 ppm. The drying can be performed by commonly known means to a person skilled in the art such as e.g. using a fluidized bed dryer or a conventional oven. The drying time can be easily adjusted by a person skilled in the art and is usually carried out over a period of 3 to 40h such as about 8 to 20h and in particular about 8 to 10h.

In all embodiments of the present invention the highly porous cross-linked polymethylmethacrylate beads are preferably prepared by suspension polymerisation of a monomer mixture consisting of 10 - 90 wt.-% methyl methacrylate and 10 - 90 wt.-% ethylene glycol dimethacrylate, with the proviso that the sum of monomers sums up to 100 wt.-%, in the presence of a porogen selected from toluene, n-hexanone, methylisobutyl ketone and isoamyl alcohol and a stabilizer selected from the group consisting of polyvinyl pyrrolidone, polyvinylmethylether, polyethyleneimine, poly(acrylicacid), polyvinylalcohol, vinyl acetate copolymer and ethyl cellulose.

Particularly suitable highly porous cross-linked polymethylmethacrylate beads according to the present invention have a Dᵥ50 selected in the range of 9 to 12 µm and an oil absorption capacity selected in the range of 1.5-2.0 cc/g. Furthermore, it is preferred that the residual monomer content is less than 100 ppm, more preferably less than 50ppm (determined by Gas Chromatography). It is furthermore advantageous if the beads exhibit as 10% aqueous dispersion in distilled water a pH in the range of 5.0 to 9.0. It is furthermore preferred if the highly porous cross-linked polymethylmethacrylate beads have a water content of less than 1.5 wt.-% (determined by Karl Fischer titration).

Suitable highly porous cross-linked polymethylmethacrylate beads according to the present invention are e.g. commercially available as VALVANCE™ *Touch* 150 at DSM Nutritional Products Ltd Kaiseraugst.

Hydrophilic benzimidazole type UVB filter substances, such as 2-phenylbenzimidazol-sulphonic acid or 2,2'-(1,4-phenylene)bis-1*H*-benzimidazole-5,7-disulfonic acid as well as the respective salts thereof are well known to a person skilled in the art. Suitable salts of 2-phenylbenzimidazol-sulphonic acid or 2,2'-(1,4-phenylene)bis-1*H*-benzimidazole-5,7-disulfonic acid are alkali salts, such as sodium- or potassium salts, ammonium salts, morpholine salts, salts of primary, sec. and tert. amines like monoethanolamine salts, diethanolamine salts and salts of basic amino acids such as arginine, lysine, ornithine and histidine and the like. The salts can be used as such or prepared in situ by neutralizing 2-phenylbenzimidazol-sulphonic acid, respectively 2,2'-(1,4-phenylene)bis-1*H-*benzimidazole-5,7-disulfonic acid in the aqueous phase during the process of preparation of the topical composition.

Preferred hydrophilic benzimidazole type UVB filter substance according to the present invention are 2-phenylbenzimidazol-sulphonic acid [CAS: 27503-81-7] or the disodium salt of 2,2'-(1,4-phenylene)bis-1*H*-benzimidazole-5,7-disulfonic acid [CAS: 180898-37-7]. Most preferred in all embodiments of the present invention is 2-phenylbenzimidazol-sulphonic acid.

Organopolysiloxane functionalized with at least one UV-light absorbing group are known as UV-filter agents and are prepared by hydrosilylation, i.e. the addition of Si-H bonds to an unsaturated carbon-carbon bond attached to a UV-light absorbing substance in the presence of a hydrosilylation catalyst such as e.g. disclosed in WO92/20690, EP1142930 or EP358584.

Preferably, the organopolysiloxanes functionalized with at least one UV-light absorbing group according to the invention always comprise at least one unit selected from the formula (Ia), (Ib), (Ic) and/ or (Id) 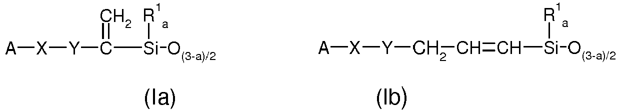 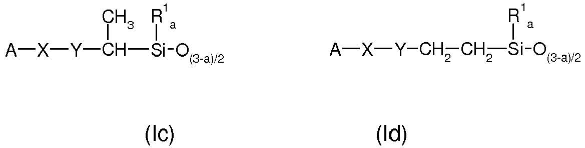 and, optionally, one or several units of formula (II) 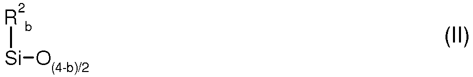 wherein
- a: is 0, 1 or 2,
- b: is 0, 1, 2, 3;
- R¹: is a C₁-C₃₀hydrocarbon group or a trimethylsilyloxy group and
- R²: is hydrogen, a C₁-C₃₀hydrocarbon group or a trimethylsilyloxy group,
- Y: is a divalent C₁-C₁₀ alkylene or C₂-C₁₀ alkenylene chain, preferably a C₁-C₄ alkylene chain, most preferably a methylene group
- X: is O, NH or NR³ wherein R³ is a C₁-C₃₀, in particular a C₁-C₃ hydrocarbon group and
- A: is a UV-B light absorbing group, a UV-A light absorbing group, a UV-C light absorbing group or a broadband light absorbing group;

The organopolysiloxane functionalized with at least one UV-light absorbing group according to the invention are polymeric materials which may be homopolymers consisting only of units of formula (Ia), (Ib), (Ic) and/ or (Id), or they may be copolymers containing units of formula (Ia), (Ib), (Ic) and/ or (Id) as well as units of formula (II). The units of formula (Ia), (Ib), (Ic) and/ or (Id) may be distributed randomly in the organopolysiloxane, they may be the end blocking units of the polymer or they may be located at the end of the polymer and pending in a chain of the polymer at the same time.

The organopolysiloxane functionalized with at least one UV-light absorbing group may carry only one kind of UV-light absorbing group or may carry at least two units of formula (Ia), (Ib), (Ic) and/ or (Id) wherein the UV-light absorbing groups are different such as e.g. a UV-B light absorbing group and a UV-A light absorbing group.

If a is 2 the two substituents R¹ may be identical or different. If b is 2 or 3 the two or three substituents R² may be identical or different. If the polymer contains more than one unit of formula (Ia), (Ib), (Ic) and/ or (Id) the substituents R¹ may be identical or different from unit to unit. If the polymer contains more than one unit of formula (II) the substituents R² may be identical or different from unit to unit.

The organopolysiloxanes functionalized with at least one UV-light absorbing group according to the invention may be linear, cyclic, branched or cross-linked. In a particular embodiment the organopolysiloxanes are linear or cyclic organopolysiloxane, characterized in that in the majority of units (Ia), (Ib), (Ic), (Id) and (II) a = 1 and b = 2. However, if the organopolysiloxane is a linear polymer at least two end blocking units must be present, thus requiring either the presence of two units in which a has a value of 2 or two units in which b is 3. Such organopolysiloxanes generally exhibit a statistical distribution of polymer chain sizes.

In another subembodiment of the invention, the organopolysiloxanes functionalized with at least one UV-light absorbing group are linear organopolysiloxanes comprising one end blocking unit of formula (IIIa) and one end blocking unit of formula (IIIb) [corresponding to units of formula (Ia), (Ib), (Ic) and/ or (Id), wherein a = 2, respectively (II), wherein b = 3] 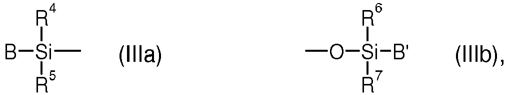 s units selected from the group of (Ia), (Ib), (Ic) and/ or (Id) as depicted above wherein a = 1 and,
r units of formula (IV) [corresponding to unit of formula (II), wherein b = 2] 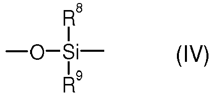 wherein
- s: is an integer from 0 to 50,
- r: is an integer from 0 to 200; and
- R¹, R⁴, R⁵, R⁶, R⁷: independently are as defined above for R¹;
- R⁸ and R⁹: independently are as defined above for R²
- B and B': independently are a group R¹ or a UV-light absorbing group selected from 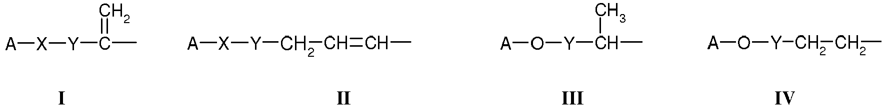
with the proviso that when s is 0 at least B or B' is a group I, II, III or IV.

In a yet other subembodiment, the organopolysiloxanes functionalized with at least one UV-light absorbing group are linear organopolysiloxanes consisting of one unit of formula (IIIa) and one unit of formula (IIIb), 4 to 10, preferably 4 to 6 units of formula (Ia) and/ or (Ib) and 40 to 90, preferably 60 to 80 units of formula (IV), wherein R¹, R⁴, R⁵, R⁶, R⁷, R⁸, R⁹, B and B' are methyl, Y is methylene and X is O, and wherein the units of formula (Ia) and/ or (Ib) are randomly distributed in the organopolysiloxanes chain. It is well understood to a person skilled in the art that the above description of the organopolysiloxanes refers to an average statistical distribution of polymer chain sizes which might still contain as minor impurities H-siloxane unit deriving from the preparation process.
Due to the preparation of the organopolysiloxanes functionalized with at least one UV-light absorbing group by hydrosilylation, i.e. the addition of a H-siloxane unit of an organopolysiloxane to an unsaturated carbon-carbon bond of a UV-light absorbing group, normally units of formula (Ia) as well as (Ib), respectively (Ic) as well as (Id) are formed and thus present at the same time in the organopolysiloxanes functionalized with at least one UV-light absorbing group. In a particular embodiment the ratio of units of formula (Ia) to units of formula (Ib) in the organopolysiloxanes functionalized with at least one UV-light absorbing group is about 4 to 1.

The term C₁-C₁₀ alkylene used according to the invention includes straight chain or branched saturated hydrocarbon residues such as methylene, 1-ethylene, 2-ethylene, 3-propylene, 2-propylene, 2-methyl-3-propylene, 3-butylene, 4-butylene, 4-pentylene, 5-pentylene, 6-hexylene, and the like.

The term C₂-C₁₀ alkenylene used according to the invention includes unsaturated hydrocarbon residues containing at least one double bond, such as for example, 2-propen-2-ylene, 2-propen-3-ylene, 3-buten-3-ylene, 3-buten-4-ylene, 4-penten-4-ylene, 4-penten-5-ylene, (3-methyl)-penta-2,4-dien-4 or 5-ylene, 11-dodecen-11-ylene, and the like. The divalent alkylene or alkenylene chains may be interrupted by one or several oxygen atoms.

The term C₁-C₃₀hydrocarbon group used according to the invention refers to saturated or unsaturated C₁-C₃₀hydrocarbon groups such as C₁-C₃₀alkyl such as methyl, ethyl, propyl and butyl; C₂-C₃₀alkenyl such as vinyl and allyl; and unsubstituted or substituted aryl such as phenyl, alkaryl and alkoxyphenyl. The hydrocarbon group is unsubstituted or substituted by, e.g. halogen, e.g. a halogenated C₁-C₁₈hydrocarbon group. The alkyl and alkenyl groups may be straight chain or branched such as e.g. methyl, ethyl, 3-propyl, 2-propyl, 2-methyl-3-propyl, 3-butyl, 4-butyl, 4-pentyl, 5-pentyl, 6-hexyl, 2-propen-2-yl, 2-propen-3-yl, 3-buten-3-yl, 3-buten-4-yl, 4-penten-4-yl, 4-penten-5-yl, (3-methyl)-penta-2,4-dien-4 or 5-yl, 11-dodecen-11-yl.

The term UV-light absorbing groups refers to all groups which absorb light in the range of wavelengths 400 - 320 nm (UVA) and 320 - 290 (UVB) or of even shorter wavelengths (UVC) and which are or can be used as chemical UV filters. The term "broadband light absorbing group" as used herein refers to groups which absorb light in a region overlapping UV-A and UV-B, especially in the range between about 310 and 360nm. Particular suitable UV-light absorbing groups which may be present in the functionalized organopolysiloxanes of the instant invention belong to the groups of acrylates, p-aminobenzoates, camphor derivatives (such as of benzylidene camphor type), cinnamates, benzophenones, esters of benzalmalonic acid, esters of 2-(4-ethoxy anilinomethylene)propandioic acid, imidazole derivatives, salicylates, triazone derivatives, triazol derivatives, dibenzoylmethanes, anthranilates, amino substituted hydroxybenzophenones, phenyl-benzoxazoles, and 1,4-dihydropyranes.

Examples of preferred UV-light absorbing groups A are those comprising a benzalmalonate, a benzoxazol, a benzylidene camphor, a benzimidazole, a dibenzoylmethane, a p-amino benzoic acid, a benzotriazol, a diphenylacrylate or a dialkylamino substituted hydroxybenzophenone group.

In a particular embodiment the UV-light absorbing group A is a 2-(4-diethylamino-2-hydroxybenzoyl)benzoyloxymethyl group. The preparation of such organosilicone derivatives of amino hydroxybenzophenones is e.g. disclosed in EP1494642 or EP1981895.

In another particular embodiment, the organopolysiloxane functionalized with at least one UV-light absorbing group comprises two different groups A which are selected from 4-[(2,2-diethoxycarbonyl)vinyl]phenoxymethyl and 2-(4-diethylamino-2-hydroxybenzoyl)benzoyloxymethyl groups. The preparation of such organopolysiloxanes functionalized with two different UV-light absorbing group is e.g. disclosed in EP1885769.

In another particular embodiment, the UV-light absorbing group A is diethylbenzalmalonate group. Thus, particularly preferred are linear organopolysiloxanes wherein the UV-light absorbing group A is diethylbenzalmalonate and which consist of one unit of formula (IIIa) and one unit of formula (IIIb), 4 to 6 units of formula (Ia) and/ or (Ib) and 60 to 80 units of formula (IV), wherein R¹, R⁴, R⁵, R⁶, R⁷, R⁸, R⁹, B and B' are methyl, Y is methylene and X is O and the units of formula (Ia) and/ or (Ib) are randomly distributed in the organopolysiloxanes chain. Even more preferably the ratio of units of formula (Ia) to units of formula (Ib) in such linear organopolysiloxanes is additionally selected in the range of about 4 to 1. Such an organopolysiloxane is e.g. commercially available as PARSOL^{®} SLX at DSM Nutritional Product Ltd Kaiseraugst (INCI polysilicone-15/ CAS: 207574-74-1).

In all embodiments of the present invention, preferably the hydrophilic benzimidazole type UVB filter is 2-phenylbenzimidazol-sulphonic acid and the organopolysiloxane functionalized with at least one UV-light absorbing group is polysilicone-15.

The concentration of the hydrophilic benzimidazole type UVB filter or a salt thereof in the topical composition according to the invention is preferably selected in the range of about 0.1 to 5 wt.-%, more preferably in the range of about 0.5 to 4 wt.-%, most preferably in the range of 1 to 3 wt.-% based on the total weight of the topical composition.

The concentration of the organopolysiloxane functionalized with at least one UV-light absorbing group in the topical composition according to the invention is preferably selected in the range of about 0.1 to 15 wt.-%, more preferably in the range of about 0.5 to 10 wt.-%, most preferably in the range of 1 to 5 wt.-% based on the total weight of the topical composition.

The term "topical" is understood here to mean external application to keratinous substances, which are in particular the skin, scalp, eyelashes, eyebrows, nails, mucous membranes and hair.

As the compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term cosmetically acceptable carrier refers to all carriers and/or excipients and/or diluents conventionally used in cosmetic compositions.

Preferred topical compositions according to the invention are skin care preparations or functional preparations.

Examples of skin care preparations are, in particular, light protective preparations (sunscreens), anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), as well as skin lightening preparations as well as BB and CC Creams.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment the topical compositions according to the invention are skin care preparations, such as (body) milks, lotions, hydrodispersions, foundations, creams, creamgels, serums, toners or gels.

The topical compositions according to the present invention may be in the form of a suspension or dispersion in solvents or fatty substances, or alternatively in the form of an emulsion or micro emulsion (in particular of oil-in-water (O/W) or water-in-oil (W/O)type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

In one embodiment, the topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the topical composition according to the invention is an O/W emulsion, then it contains advantageously at least one O/W- or Si/W-emulsifier selected from the list of PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, Glyceryl Stearate Citrate, Glyceryl Stearate SE (self-emulsifying), stearic acid, salts of stearic acid, polyglyceryl-3-methylglycosedistearate. Further suitable emulsifiers are phosphate esters and the salts thereof such as cetyl phosphate (Amphisol^{®} A), diethanolamine cetyl phosphate (Amphisol^{®}DEA), potassium cetyl phosphate (Amphisol^{®} K), sodiumcetearylsulfat, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, Cetearyl Glucoside, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

The at least one O/W, respectively Si/W emulsifier is preferably used in an amount of 0.5 to 10 wt.-%, in particular in the range of 0.5 to 6 wt.-% such as more in particular in the range of 0.5 to 5 wt.-% such as most in particular in the range of 1 to 4 wt.-%, based on the total weight of the composition.

Particular suitable O/W emulsifiers according to the present invention encompass phosphate esters emulsifier of formula (II) 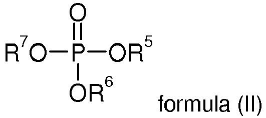 wherein R⁵, R⁶ and R⁷ may be hydrogen, an alkyl of from 1 to 22 carbons, preferably from 12 to 18 carbons; or an alkoxylated alkyl having 1 to 22 carbons, preferably from 12 to 18 carbons, and having 1 or more, preferably from 2 to 25, most preferably 2 to 12, moles ethylene oxide, with the provision that at least one of R⁵, R⁶ and R⁷ is an alkyl or alkoxylated alkyl as previously defined but having at least 6 alkyl carbons in said alkyl or alkoxylated alkyl group.

Monoesters in which R⁵ and R⁶ are hydrogen and R⁷ is selected from alkyl groups of 10 to 18 carbons and alkoxylated fatty alcohols of 10 to 18 carbons and 2 to 12 moles ethylene oxide are preferred. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate.

A particular O/W emusifier to be used in the topical compositions according to the invention is potassium cetyl phosphate e.g. commercially available as Amphisol^{®} K at DSM Nutritional Products Ltd Kaiseraugst.

Further suitable O/W emulsifiers are polyethyleneglycol (PEG) esters or diesters such as e.g. [INCI Names] PEG-100 Stearate, PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate.
Particularly preferred according to the present invention is PEG-100 Stearate sold under the tradename Arlacel™ 165 (INCI Glyceryl Stearate (and) PEG-100 Stearate) by Croda.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

In particular embodiment, the invention relates to topical compositions with all the definitions and preferences given herein in the form of O/W emulsions comprising an oily phase dispersed in an aqueous phase in the presence of an O/W emulsifier wherein the O/W emulsifier is selected from the group consisting of potassium cetyl phosphate, glyceryl stearate (and) PEG-100 Stearate, cetearyl olivate and sorbitan olivate as well as mixtures thereof.

In another particular embodiment, the invention relates to topical compositions in the form of W/O emulsions comprising an aqueous phase dispersed in an oily phase in the presence of a W/O emulsifier.

Suitable W/O emulsifiers encompasse polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Particularly preferred according to the present invention are W/O emulsions wherein the W/O emulsifier is Polyglyceryl-3 Diisostearate.

The topical compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 5 wt.-%, such as most in particular in the range of 1 to 3 wt.-%, based on the total weight of the composition. Particular suitable co-surfactants are selected from the list of alkyl alcohols such as cetyl alcohol (Lorol C16, Lanette 16) cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof.

The compositions in form of O/W or W/O emulsions according to the invention can be provided, for example, in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

According to an advantageous embodiment of the invention the topical compositions constitute cosmetic composition and are intended for topical application to the skin.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions of the invention can also contain usual cosmetic adjuvants and additives, such as preservatives/antioxidants, fatty substances/oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into such compositions. Such cosmetic ingredients commonly used in the skin care industry, which are suitable for use in the compositions of the present invention, are e.g. described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Preferably, the topical compositions according to the invention comprise further UV filter substances which are preferably selected from conventional UVA and/or UVB and/ or broad spectrum UV-filter substances known to be added into topical compositions such as cosmetic or dermatological sun care products. Such UV-filter substances comprise all groups which absorb light in the range of wavelengths 400 nm to 320 nm (UVA) and 320 nm to 280 nm (UVB) or of even shorter wavelengths (UVC) and which are or can be used as cosmetically acceptable UV-filter substances. Such UV-filter substances are e.g. listed in International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Suitable UV-filter substances may be organic or inorganic compounds. Exemplary organic UV-filter substances encompass e.g. acrylates such as e.g. 2-ethylhexyl 2-cyano-3,3-diphenylacrylate (octocrylene, PARSOL^{®} 340), ethyl 2-cyano-3,3-diphenylacrylate; Camphor derivatives such as e.g. 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, terephthalylidene dicamphor sulfonic acid (Mexoryl^{®} SX); Cinnamate derivatives such as e.g. ethylhexyl methoxycinnamate (PARSOL^{®} MCX), ethoxyethyl methoxycinnamate, isoamyl methoxycinnamate as well as cinnamic acid derivatives bond to siloxanes; p-Aminobenzoic acid derivatives such as e.g. p-aminobenzoic acid, 2-ethylhexyl p-dimethylaminobenzoate, N-oxypropylenated ethyl p-aminobenzoate, glyceryl p-aminobenzoate; Benzophenones such as e.g. benzophenone-3, benzophenone-4, 2,2',4,4'-tetrahydroxy-benzophenone, 2,2'-dihydroxy-4,4'-dimethoxybenzophenone; Esters of benzalmalonic acid such as e.g. di-(2-ethylhexyl) 4-methoxybenzalmalonate; Organosiloxane compounds carrying chromophore groups such as e.g. polysilicones-15 (PARSOL^{®} SLX), drometrizole trisiloxane (Mexoryl^{®} XL); Salicylate derivatives such as e.g. isopropylbenzyl salicylate, benzyl salicylate, butyl salicylate, ethylhexyl salicylate (PARSOL^{®} EHS, Neo Heliopan^{®} OS), isooctyl salicylate or homomenthyl salicylate (homosalate, PARSOL^{®} HMS, Neo Heliopan^{®} HMS); Triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S); Benzotriazole derivatives such as e.g. 2,2'-methylene-bis-(6-(2H-benzotriazole-2-yl)-4-(1,1,3,3,-tetramethylbutyl)-phenol (Tinosorb^{®} M); Encapsulated UV-filters such as e.g. encapsulated ethylhexyl methoxycinnamate (Eusolex^{®} UV-pearls) or microcapsules loaded with UV-filters as e.g. dislosed in EP 1471995; Dibenzoylmethane derivatives such as e.g. 4-tert.-butyl-4'-methoxydibenzoyl-methane (PARSOL^{®} 1789), dimethoxydibenzoylmethane, isopropyldibenzoylmethane; Amino substituted hydroxybenzophenones such as e.g. 2-(4-diethylamino-2-hydroxy-benzoyl)-benzoic acid hexylester (Aminobenzophenon, Uvinul^{®} A Plus); Benzoxazol-derivatives such as e.g. 2,4-bis-[5-1(dimethylpropyl)benzoxazol-2-yl-(4-phenyl)-imino]-6-(2-ethylhexyl)-imino-1,3,5-triazine (Uvasorb^{®} K2A);
Inorganic UV-filter substances encompass pigments such as e.g. microparticulated Zink oxide or Titanium dioxide (e.g. commercially available as PARSOL^{®} TX) The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.
In order to enhance the photostability of sun care products it may be desirable to add a photostabilizer. Exemplary photostabilizers known to a skilled person in the art encompass e.g. 3,3-diphenylacrylate derivatives such as e.g. octocrylene (PARSOL^{®} 340) or Polyester-8 (Polycrylene^{®}); Benzylidene camphor derivatives such as e.g. 4-methyl benzylidene camphor (PARSOL^{®} 5000); Dialkyl naphthalates such as diethylhexyl naphthalate (Corapan TQ) without being limited thereto. An overview on further stabilizers is e.g. given in 'SPF Boosters & Photostability of Ultraviolet Filters', HAPPI, October 2007, p. 77-83 which is included herein by reference. The photostabilizers are generally used in an amount of 0.05 to 10 wt.-% with respect to the total weigh of the topical composition.

Generally, the amount of each UV-filter substance in the topical compositions according to the invention is selected in the range of about 0.1 to 10 wt.-%, preferably in the range of about 0.2 to 7 wt.-%, most preferably in the range of about 0.5 to 5 wt.-% with respect to the total weigh of the topical composition.

The total amount of UVA-filter substance(s), in particular of butyl methoxydibenzoylmethane, in the topical compositions according to the invention is preferable selected in the range of about 2 to 8 wt.-%, in particular in the range of about 2.5 to 6 wt.-%, most particular in the range of about 3 to 5 wt.-% with respect to the total weight of the topical composition.

The total amount of UV-filter substances in the topical compositions according to the invention is preferably in the range of about 1 to 40 wt.-%, preferably in the range of about 5 to 30 wt.-%, in particular in the range of 20 to 30 wt.-% with respect to the total weight of the topical composition.

Preferred further UVB-filter substances to be used in the topical compositions according to the invention encompass octocrylene, ethylhexyl methoxycinnamate, ethyl hexylsalicylate and/ or homosalate.

Preferred broadband UV-filter substances to be used in the topical compositions according to the invention encompass unsymmetrical s-triazine derivatives such 2,4-Bis-{[4-(2-ethyl-hexyloxy)-2-hydroxy]-phenyl}-6-(4-methoxyphenyl)-1,3,5-triazin, certain benzophenones such as e.g. 2-Hydroxy-4-methoxy-benzophenon, 2,2'-Methylen-bis-(6-(2H-benzotriazol-2-yl)-4-(1,1,3,3-tetramethyl-butyl)-phenol), and/ or titanium dioxide.

The preferred UVA-filter substance to be used in the topical compositions according to the invention is butyl methoxydibenzoylmethane.

In a particular preferred embodiment the present invention relates to topical compositions according to the invention comprising at least one additional UV-filter selected from the group of butyl methoxydibenzoylmethane, octocrylene, homosalate or titanium dioxide as well as mixtures thereof. In this case, it is further preferred if the amount of the 2-phenylbenzimidazol-sulphonic acid is selected in the range of 1-3 wt.-%, the amount of polysilicone-15 is selected in the range of 1-4 wt.-%, the amount of butyl methoxydibenzoylmethane is selected in the range of 3-5 wt.-%, the amount of octocrylene is selected in the range of 6-10 wt.-%, the amount of homosalate is selected in the range of 2-5 wt.-% and the amount of titanium dioxide is selected in the range of 0.5-2 wt.-%.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range of 3 to 10, preferably a pH in the range of 4 to 8 and most preferably a pH in the range of 4 to 7.5. The pH can easily be adjusted as desired with suitable acids such as e.g. citric acid or bases such as sodium hydroxide (e.g. as aqueous solution), Triethanolamine (TEA Care), Tromethamine (Trizma Base) and Aminomethyl Propanol (AMP-Ultra PC 2000) according to standard methods in the art.

The amount of the topical composition to be applied to the skin is not critical and can easily be adjusted by a person skilled in the art. Preferably the amount is selected in the range of 0.1 to 3 mg/ cm² skin, such as preferably in the range of 0.1 to 2 mg/ cm² skin and most preferably in the range of 0.5 to 2 mg / cm² skin.

The following examples are provided to further illustrate the compositions and effects of the present invention. These examples are illustrative only and are not intended to limit the scope of the invention in any way.

### Example:

### 1. In vitro SPF measurements

In vitro SPF method: application of 1.3 mg/cm², of the respective preparation on 3 Schönberg plates: roughness: 5 µm; measurement of 9 points per plate; Labsphere UV Transmittance Analyzer 2000.

**Table 1**

| | **Trade name** | **INCI Name** | **1** (Placebo) | **2** (Sample) | **Ref 1** (Placebo) | **Ref 2** (Sample) |
|---|---|---|---|---|---|---|
| | | | **Wt.-%** | **Wt.-%** | **Wt.-%** | **Wt-%** |
| A | PARSOL^{®} 1789 | Butyl methoxydibenzoylmethane | | | 4.00 | 4.00 |
| A | PARSOL^{®} 340 | Octocrylene | | | 8.00 | 8.00 |
| **A** | **PARSOL^{®} SLX** | **Polysilicone-15** | **3.00** | **3.00** | | |
| A | AMPHISOL^{®} K | Potassium cetyl phosphate | 3.00 | 3.00 | 3.00 | 3.00 |
| A | Cetiol MM | Myristyl myristate | 1.50 | 1.50 | 1.50 | 1.50 |
| A | Lanette O | Cetearyl alcohol | 2.00 | 2.00 | 2.00 | 2.00 |
| A | Butylated Hydroxytoluene | BHT | 0.05 | 0.05 | 0.05 | 0.05 |
| A | Phenonip | Phenoxyethanol; | 0.80 | 0.80 | 0.80 | 0.80 |
| | | Methylparaben; | | | | |
| | | Ethylparaben; | | | | |
| | | Butylparaben; | | | | |
| | | Propylparaben; | | | | |
| | | Isobutylparaben | | | | |
| A | Dermofeel^{®} BGC | Butylene glycol dicaprylate/dicaprate | 8.00 | 8.00 | 8.00 | 8.00 |
| A | Finsolv TN | C12-15 alkyl benzoate | 4.00 | 4.00 | 4.00 | 4.00 |
| A | Antaron V-220 | VP/eicosene copolymer | 0.50 | 0.50 | 0.50 | 0.50 |
| A | Cetiol CC | Dicaprylyl carbonate | 4.00 | 4.00 | 4.00 | 4.00 |
| B | Glycerin 1,23 (86.5%) Ph. Eur. | Glycerin | 3.00 | 3.00 | 3.00 | 3.00 |
| B | Edeta BD | Disodium EDTA | 0.10 | 0.10 | 0.10 | 0.10 |
| B | WATER DEM. | Aqua | Ad 100 | Ad 100 | Ad 100 | Ad 100 |
| C | Keltrol CG-T | Xanthan gum | 0.30 | 0.30 | 0.30 | 0.30 |
| D | Triethanolamine Care | Triethanolamine | 0.30 | 0.30 | | |
| E | WATER DEM. | Aqua | 5.00 | 5.00 | | |
| E | Triethanolamine | Triethanolamine | 1.80 | 1.80 | | |
| **E** | **PARSOL^{®} HS** | **Phenylbenzimidazol sulfonic acid** | **3.00** | **3.00** | | |

| **F** | **VALVANCE *Touch* 150** | **Methyl Methacrylate Cross Polymer** | | **3.00** | | **3.00** |
|---|---|---|---|---|---|---|
| | **In vitro SPF** | | **4** | **5** | **28** | **26** |
| | **Delta*** | | | **+25%** | | **-7%** |

| | | | | | | |
|---|---|---|---|---|---|---|
| *([Value sample - value Placebo)]/ value Placebo)*100% | | | | | | |

As can be retrieved, the in vitro SPF of the preparations according to the present invention comprising as UV-filter the combination of Polysilicone-15 and Phenylbenzimidazol sulfonic acid is significantly enhanced through the addition of the beads, whereas the in vitro SPF of the reference samples (Ref1 and Ref2) comprising as UV-filter the combination Butyl methoxydibenzoylmethane and Octocrylene did not increase but even slightly decreased.

### 2. In vivo SPF measurements

The in-vivo SPF of several O/W emulsions according to the present invention has been tested at an external test institute (Dermaconsult Concept GmbH, Bonn, Germany) according to the *COLIPA International Sun Protection Factor Test Method.* The results are presented in tables 2 and 3.

**Table 2: O/W Emulsion**

| | **Trade name** | **INCI Name** | 3 (Placebo) | 4 (Sample) | 5 (Sample) | 6 (Sample) |
|---|---|---|---|---|---|---|
| | | | **Wt.-%** | **Wt.-%** | **Wt.-%** | **Wt.-%** |
| A | PARSOL^{®} 1789 | Butyl methoxydibenzoylmethane | 4.00 | 4.00 | 4.00 | 4.00 |
| **A** | **PARSOL^{®} SLX** | **Polysilicone-15** | **2.00** | **2.00** | **2.00** | **2.00** |
| A | PARSOL^{®} 340 | Octocrylene | 8.00 | 8.00 | 8.00 | 8.00 |
| A | AMPHISOL^{®} K | Potassium cetyl phosphate | 3.00 | 3.00 | 3.00 | 3.00 |
| A | Lanette O | Cetearyl alcohol | 2.00 | 2.00 | 2.00 | 2.00 |
| A | Butylated Hydroxytoluene | BHT | 0.05 | 0.05 | 0.05 | 0.05 |
| A | Phenonip | Phenoxyethanol; | 0.80 | 0.80 | 0.80 | 0.80 |
| | | Methylparaben; | | | | |
| | | Ethylparaben; | | | | |
| | | Butylparaben; | | | | |
| | | Propylparaben; | | | | |
| | | Isobutylparaben | | | | |
| A | Dermofeel^{®} BGC | Butylene glycol dicaprylate/dicaprate | 8.00 | 8.00 | 8.00 | 8.00 |
| A | Finsolv TN | C12-15 alkyl benzoate | 4.00 | 4.00 | 4.00 | 4.00 |
| A | Antaron V-220 | VP/eicosene copolymer | 0.50 | 0.50 | 0.50 | 0.50 |
| A | Cetiol CC | Dicaprylyl carbonate | 4.00 | 4.00 | 4.00 | 4.00 |
| A | Cetiol MM | Myristyl myristate | 1.50 | 1.50 | 1.50 | 1.50 |
| A | PARSOL^{®} HMS | Homosalate | 4.00 | 4.00 | 4.00 | 4.00 |
| B | Glycerin 1,23 (86.5%) Ph. Eur. | Glycerin | 3.00 | 3.00 | 3.00 | 3.00 |
| B | Edeta BD | Disodium EDTA | 0.10 | 0.10 | 0.10 | 0.10 |
| B | WATER DEM. | Aqua | Ad 100 | Ad 100 | Ad 100 | Ad 100 |
| C | Keltrol CG-T | Xanthan gum | 0.30 | 0.30 | 0.30 | 0.30 |
| D | Triethanolamine Care | Triethanolamine | 0.30 | 0.30 | 0.30 | 0.30 |
| E | WATER DEM. | Aqua | 5.00 | 5.00 | 5.00 | 5.00 |
| E | Triethanolamine | Triethanolamine | 1.20 | 1.20 | 1.20 | 1.20 |
| **E** | **PARSOL^{®} HS** | **Phenylbenzimidazol sulfonic acid** | **2.00** | **2.00** | **2.00** | **2.00** |
| **F** | **VALVANCE™ *Touch 210*** | **Silica** | | **3.00** | | |
| **F** | **VALVANCE™ *Touch* 250** | **Silica, methicone** | | | **3.00** | |
| **F** | **VALVANCE™ *Touch 150*** | **Methyl Methacrylate Cross polymer** | | | | **1.00** |
| | **In vivo SPF** | | **39** | **60** | **62** | **49** |
| | **Delta*** | | | **+54%** | **+59%** | **+26%** |

| | | | | | | |
|---|---|---|---|---|---|---|
| *([Value sample - value Placebo)]/ value Placebo)*100% | | | | | | |

**Table 3: O/W Emulsion**

| **Trade name** | **INCI name** | 7 Placebo | 8 Sample |
|---|---|---|---|
| | | Wt.-% | Wt.-% |
| PARSOL^{®} 1789 | Butyl Methoxydibenzolylmethane | 3.0 | 3.0 |
| PARSOL^{®} 340 | Octocrylene | 4.0 | 4.0 |
| **PARSOL^{®} SLX** | **Polysilicone-15** | **1.0** | **1.0** |
| PARSOL^{®} TX | Titanium Dioxide | 1.0 | 1.0 |
| DUB DIS | Diisopropyl Sebacate | 6.0 | 6.0 |
| Finsolv TN | C12-15 Alkyl Benzoate | 5.0 | 5.0 |
| Amphisol^{®} K | Potassium Cetyl Phospate | 3.0 | 3.0 |
| Lanette O | Cetearyl Alcohol | 2.5 | 2.5 |
| Lanette 22 | Behenyl Alcohol | 2.5 | 2.5 |
| Carbopol Ultrez 21 | Acrylates/C10-30 Alkyl Acrylate Crosspolymer | 0.10 | 0.10 |
| Euxyl PE 9010 | Phenoxyethanol & Ethylhexylglycerin | 1.0 | 1.0 |
| Keltrol CG-T | Xanthan Gum | 0.3 | 0.3 |
| Edeta | Disodium Edta | 0.1 | 0.1 |
| Butylene Glycol | Butylene Glycol | 2.0 | 2.0 |
| Water | Aqua | Ad 100 | Ad 100 |
| **PARSOL^{®} HS** | **Phenylbenzimidazole Sulfonic Acid** | **1.5** | **1.5** |
| Water | Aqua | 10.0 | 10.0 |
| Trisma Base | Tromethamine | 0.9 | 0.9 |
| **VALVANCE™ *Touch150*** | **Methyl Methacrylate Cross polymer** | | **3.0** |
| **In vivo SPF** | | **16.2** | **28.5** |
| **Delta*** | | | **+76%** |

| | | | |
|---|---|---|---|
| *([Value sample - value Placebo)]/ value Placebo)*100% | | | |

As can be retrieved, the in vivo SPF of the preparations according to the present invention was significantly increased compared to the placebo without the respective beads.

### Reference Experiments

The in-vivo SPF of several O/W emulsions which did not comprise a UV-filter combination according to the present invention (i.e a hydrophilic benzimidazole type UVB filter and an organopolysiloxane functionalized with at least one UV-light absorbing group) has been tested at an external test institute (Dermaconsult Concept GmbH, Bonn, Germany) according to the *COLIPA International Sun Protection Factor Test Method.* The results are presented in tables 4 and 5.

**Table 4: O/W Emulsion**

| **Trade name** | **INCI name** | 9 Placebo | 10 Ref. | 11 Ref. | 12 Ref. |
|---|---|---|---|---|---|
| | | Wt.-% | Wt.-% | Wt.-% | Wt.-% |
| PARSOL^{®} 1789 | Butyl Methoxydibenzolylmethane | 4.0 | 4.0 | 4.0 | 4.0 |
| PARSOL^{®} 340 | Octocrylene | 10.0 | 10.0 | 10.0 | 10.0 |
| PARSOL^{®} EHS | Ethylhexyl salicylate | 5.0 | 5.0 | 5.0 | 5.0 |
| PARSOL^{®} HMS | Homomenthylsalicylate | 4.0 | 4.0 | 4.0 | 4.0 |
| Amphisol^{®} K | Potassium Cetyl Phospate | 2.5 | 2.5 | 2.5 | 2.5 |
| Lanette O | Cetearyl Alcohol | 1.5 | 1.5 | 1.5 | 1.5 |
| Butylated Hydroxytoluene | BHT | 0.05 | 0.05 | 0.05 | 0.05 |
| Phenonip | Phenoxyethanol, Methylparaben, Ethylparaben, Butylparaben, Propylparaben, Isobutylparaben | 0.8 | 0.8 | 0.8 | 0.8 |
| Dermofeel^{®} BGC | Butylene glycol dicarylate/dicaprate | 8.0 | 8.0 | 8.0 | 8.0 |
| Antaron V-220 | VP/Eicosene Copolymer | 0.5 | 0.5 | 0.5 | 0.5 |
| Cetiol CC | Dicaprylyl carbonate | 4.0 | 4.0 | 4.0 | 4.0 |
| Cetiol MM | Myristyl myristate | 0.5 | 0.5 | 0.5 | 0.5 |
| Glycerin 1,23 (86.5%) Ph. Eur. | Glycerin | 3.0 | 3.0 | 3.0 | 3.0 |
| Edeta BD | Disodium EDTA | 0.1 | 0.1 | 0.1 | 0.1 |
| Water dem. | Aqua | Ad 100 | Ad 100 | Ad 100 | Ad 100 |
| Pemulene TR-1 | Acrylates/C10-30 Alkyl Acrylate crosspolymer | 0.15 | 0.15 | 0.15 | 0.15 |
| Keltrol CG-T | Xanthan gum | 0.15 | 0.15 | 0.15 | 0.15 |
| PARSOL^{®} TX | Titanium dioxide; silica; dimethicone | 3.0 | 3.0 | 3.0 | 3.0 |
| Sodium hydroxide 30% soln. | Aqua, sodium hydroxide | 0.15 | 0.15 | 0.15 | 0.15 |
| **VALVANCE™ *Touch150*** | **Methyl Methacrylate Cross polymer** | | **1.0** | | |
| **VALVANCE™ *Touch 210*** | **Silica** | | | **3.0** | |
| **VALVANCE™ *Touch* 250** | **Silica, methicone** | | | | **3.0** |
| **In vivo SPF** | | 50 | 38 | 54 | 46 |
| **Delta*** | | | **-24%** | **+8%** | **-8%** |

| | | | | | |
|---|---|---|---|---|---|
| *([Value sample - value Placebo)]/ value Placebo)*100% | | | | | |

**Table 5: O/W Emulsion**

| **Trade name** | **INCI name** | 9 Placebo | 10 Ref. | 11 Ref. | 12 Ref. |
|---|---|---|---|---|---|
| | | Wt.-% | Wt.-% | Wt.-% | Wt.-% |
| PARSOL® 1789 | Butyl Methoxydibenzoylmethane | 5.00 | 5.00 | 5.00 | 5.00 |
| DL-ALPHA-TOCOPHERYL ACETAT | Tocopheryl acetate | 0.50 | 0.50 | 0.50 | 0.50 |
| Finsolv TN | C12-15 Alkyl benzoate | 5.00 | 5.00 | 5.00 | 5.00 |
| Dermofeel® BGC | Butylene glycol dicarylate/dicaprate | 4.00 | 4.00 | 4.00 | 4.00 |
| AMPHISOL® K | Potassium Cetyl Phospate | 3.00 | 3.00 | 3.00 | 3.00 |
| Lanette 16 | Cetyl alcohol | 0.80 | 0.80 | 0.80 | 0.80 |
| Lanette O | Cetearyl alcohol | 0.80 | 0.80 | 0.80 | 0.80 |
| Euxyl PE 9010 | Phenoxyethanol & Ethylhexylglycerin | 0.80 | 0.80 | 0.80 | 0.80 |
| PARSOL® 340 | Octocrylene | 14.00 | 14.00 | 14.00 | 14.00 |
| PARSOL® HMS | Homosalate | 10.00 | 10.00 | 10.00 | 10.00 |
| Antaron V-220 | VP/Eicosene Copolymer | 1.00 | 1.00 | 1.00 | 1.00 |
| Edeta BD | Disodium EDTA | 0.10 | 0.10 | 0.10 | 0.10 |
| Keltrol CG-T | Xanthan Gum | 0.20 | 0.20 | 0.20 | 0.20 |
| PARSOL® TX | Titanium dioxide; silica; dimethicone | 5.00 | 5.00 | 5.00 | 5.00 |
| Pemulen TR-1 | Acrylates/C10-30 Alkyl Acrylate crosspolymer | 0.15 | 0.15 | 0.15 | 0.15 |
| WATER DEM. | Aqua | 37.70 | 36.70 | 34.70 | 34.70 |
| Glycerine 99.5% AMI | Glycerin | 5.00 | 5.00 | 5.00 | 5.00 |
| PH. EUR. Vegetable | | | | | |
| Triethanolamine Care | Triethanolamine | 0.15 | 0.15 | 0.15 | 0.15 |
| **PARSOL® HS** | **Phenylbenzimidazol sulphonic acid** | **1.00** | **1.00** | **1.00** | **1.00** |
| Triethanolamine Care | Triethanolamine | 0.80 | 0.80 | 0.80 | 0.80 |
| WATER DEM. | Aqua | 5.00 | 5.00 | 5.00 | 5.00 |
| **Valvance™ Touch 150** | **Methyl Methacrylate Crosspolymer** | | **1.00** | | |
| **Valvance™ Touch 210** | **Silica** | | | **3.00** | |
| **Valvance™ Touch 250** | **Silica, Methicone** | | | | **3.00** |
| **In vivo SPF** | | **62** | **66** | **63** | **63** |
| **Delta*** | | | **+6%** | **+2%** | **+2%** |

As can be retrieved, the in vivo SPF of the reference samples did not result in a significant increase of the in vivo SPF or even in a decrease in the in vivo SPF.

### 3. Sensory evaluation

The samples prepared as outlined above were tested in a blind study with a trained sensorial panel consisting of 8 persons under the following conditions:
The evaluation takes part on the inner forearm; the panel leader applies 50 µL of the respective sample.

Evaluator spreads the product within a defined circle of 5 cm diameter using index or middle finger, circular motion, rate of 2 rotations/second. This is the so called rub-out phase. After the rub-out phase the after-feel is assessed relative to a set of standards. The afterfeel can have 4 different aspects: oil, grease, waxy or/and silicone.

The relative intensities felt are scaled on a scale from 0 to 100 in comparison to training standards, with the premise that all 4 aspects are calibrated to add up to 100 %. The higher a percentage the more an aspect dominates the after-feel. Data are median values. % Silicone indicates the percentage of perceived silicone character (velvety or silky).

**Table 6: Results of sensory evaluation**

| | immediate | 20 minutes |
|---|---|---|
| O/W emulsion of | % silicone in residue | % silicone in residue |
| Table 2, sample 3 (Placebo) | 13.14 | 26.50 |
| Table 2, sample 4 | 23.74 | 32.47 |
| 3% VALVANCE™ *Touch 210* | | |
| Table 2, sample 5 | 28.10 | 42.78 |
| 3% VALVANCE™ *Touch 250* | | |
| Table 2, sample 6 | 22.46 | 28.16 |
| 1% VALVANCE™ *Touch 150* | | |

As can be retrieved from table 4, the beads according to the present invention significantly improved the silicone feel directly after application as well as after 20 min compared to the placebo.

## Claims

1. A topical composition comprising a hydrophilic benzimidazole type UVB filter, an organopolysiloxane functionalized with at least one UV-light absorbing group, and highly porous silica and/or highly porous cross-linked polymethylmethacrylate beads having a particle size Dᵥ0 of greater 0.3 µm, a Dᵥ100 of less than 35 µm, a Dᵥ50 selected in the range of 9 to 15 µm and an oil absorption capacity selected in the range of 1.2-2.5 cc/g.

2. The topical composition according to claim 1, **characterized in that** the amount of the beads is independently of each other selected in the range of 0.1 - 5 wt.-% based on the total weight of the cosmetic composition.

3. The topical composition according to claim 1 or 2, **characterized in that** the composition comprises only silica beads.

4. The topical composition according to claim 3, **characterized in that** the oil absorption capacity of the silica beads is selected in the range of 1.3 to 1.8 cc/g.

5. The topical composition according to any one of claims 1 to 4, **characterized in that** the silica beads are further coated with dimethicone.

6. The topical composition according to claim 1 or 2, **characterized in that** the composition comprises only cross-linked polymethylmethacrylate beads.

7. The topical composition according to claim 6, **characterized in that** the oil absorption capacity of the cross-linked polymethylmethacrylate beads is selected in the range of 1.5 to 2.0 cc/g.

8. The topical composition according to any one of claims 1 to 7, **characterized in that** the benzimidazole type UVB filter is 2-phenylbenzimidazol-sulphonic acid and the organopolysiloxane functionalized with at least one UV-light absorbing group is polysilicone-15.

9. The topical composition according to any one of claims 1 to 8, **characterized in that** the amount of the hydrophilic benzimidazole type UVB filter is selected in the range of 0.1 to 5 wt.-% based on the total weight of the composition.

10. The topical composition according to any one of claims 1 to 9, **characterized in that** the amount of the organopolysiloxane functionalized with at least one UV-light absorbing group is selected in the range of 0.1 to 15 wt.-% based on the total weight of the composition.

11. The topical composition according to any one of claims 1 to 10, **characterized in that** the composition further comprises at least one additional UV-filter selected from the group of butyl methoxydibenzoylmethane, octocrylene, homosalate or titanium dioxide as well as mixtures thereof.

12. The topical composition according to any one of claims 1 to 11, **characterized in that** the topical composition is in the form of an oil-in-water (O/W) emulsion comprising an oily phase dispersed in an aqueous phase in the presence of an O/W emulsifier.

13. The topical composition according to claim 12, **characterized in that** the O/W emulsifier is potassium cetyl phosphate.

14. Use of highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g to enhance the SPF of a topical composition comprising a hydrophilic benzimidazole type UVB filter and an organopolysiloxane functionalized with at least one UV-light absorbing group.

15. A method of enhancing the in vivo SPF of a topical composition comprising a hydrophilic benzimidazole type UVB filter and an organopolysiloxane functionalized with at least one UV-light absorbing group, said method comprising the step of adding to the topical composition highly porous silica and/ or highly porous cross-linked polymethylmethacrylate beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g.

## Patentansprüche

1. Topische Zusammensetzung, umfassend einen hydrophilen Benzimidazol-UVB-Filter, ein mit mindestens einer UV-lichtabsorbierenden Gruppe funktionalisiertes Organopolysiloxan und hochporöse Kieselsäure und/oder hochporöse vernetzte Polymethylmethacrylatperlen mit einer Teilchengröße Dᵥ0 von größer als 0,3 µm, einer Dᵥ100 von kleiner als 35 µm, einer Dᵥ50, ausgewählt im Bereich von 9 bis 15 µm und einer Ölabsorptionskapazität, ausgewählt im Bereich von 1,2-2,5 cc/g.

2. Topische Zusammensetzung nach Anspruch 1, **dadurch gekennzeichnet, dass** die Menge der Perlen unabhängig voneinander ausgewählt ist im Bereich von 0,1-5 Gew.-%, bezogen auf das Gesamtgewicht der kosmetischen Zusammensetzung.

3. Topische Zusammensetzung nach Anspruch 1 oder 2, **dadurch gekennzeichnet, dass** die Zusammensetzung nur Kieselsäureperlen umfasst.

4. Topische Zusammensetzung nach Anspruch 3, **dadurch gekennzeichnet, dass** die Ölabsorptionskapazität der Kieselsäureperlen im Bereich von 1,3 bis 1,8 cc/g gewählt wird.

5. Topische Zusammensetzung nach einem der Ansprüche 1 bis 4, **dadurch gekennzeichnet, dass** die Kieselsäureperlen weiter mit Dimethicon überzogen sind.

6. Topische Zusammensetzung nach Anspruch 1 oder 2, **dadurch gekennzeichnet, dass** die Zusammensetzung nur vernetzte Polymethylmethacrylatperlen umfasst.

7. Topische Zusammensetzung nach Anspruch 6, **dadurch gekennzeichnet, dass** die Ölabsorptionskapazität der vernetzten Polymethylmethacrylatperlen im Bereich von 1,5 bis 2,0 cc/g gewählt ist.

8. Topische Zusammensetzung nach einem der Ansprüche 1 bis 7, **dadurch gekennzeichnet, dass** der UVB-Filter des Benzimidazol-Typs 2-Phenylbenzimidazolsulfonsäure ist und dass das Organopolysiloxan, das mit mindestens einer UV-lichtabsorbierenden Gruppe funktionalisiert ist, Polysilicon-15 ist.

9. Topische Zusammensetzung nach einem der Ansprüche 1 bis 8, **dadurch gekennzeichnet, dass** die Menge des hydrophilen UVB-Filters des Benzimidazoltyps ausgewählt ist im Bereich von 0,1-5 Gew.-%, bezogen auf das Gesamtgewicht der Zusammensetzung.

10. Topische Zusammensetzung nach einem der Ansprüche 1 bis 9, **dadurch gekennzeichnet, dass** die Menge des Organopolysiloxans, das mit mindestens einer UV-lichtabsorbierenden Gruppe funktionalisiert ist, ausgewählt ist im Bereich von 0,1-15 Gew.-%, bezogen auf das Gesamtgewicht der Zusammensetzung.

11. Topische Zusammensetzung nach einem der Ansprüche 1 bis 10, **dadurch gekennzeichnet, dass** die Zusammensetzung ferner mindestens einen zusätzlichen UV-Filter umfasst, der ausgewählt ist aus der Gruppe aus Butylmethoxydibenzoylmethan, Octocrylen, Homosalat oder Titandioxid sowie deren Mischungen.

12. Topische Zusammensetzung nach einem der Ansprüche 1 bis 11, **dadurch gekennzeichnet, dass** die topische Zusammensetzung in Form einer Öl-in-Wasser (O/W)-Emulsion vorliegt, die eine in einer wässrigen Phase dispergierte Ölphase in Gegenwart eines O/W Emulgators umfasst.

13. Topische Zusammensetzung nach Anspruch 12, **dadurch gekennzeichnet, dass** der O/W Emulgator Kaliumcetylphosphat umfasst.

14. Verwendung von hochporösen Kieselsäure- und/oder hochporösen vernetzten Polymethylmethacrylatperlen mit einer Teilchengröße Dᵥ0 von größer als 0,3 µm, Dᵥ100 von kleiner als 35 µm, Dᵥ50 ausgewählt im Bereich von 9 bis 15 µm und einer Ölabsorptionskapazität ausgewählt im Bereich von 1,2-2,5 cc/g, zur Steigerung des SPF einer topischen Zusammensetzung, umfassend einen hydrophilen Benzimidazol-UVB-Filter und ein Organopolysiloxan, das mit mindestens einer UV-Licht absorbierenden Gruppe funktionalisiert ist.

15. Verfahren zur Steigerung des in-vivo SPF einer topischen Zusammensetzung, umfassend einen hydrophilen Benzimidazol-UVB-Filter und ein mit mindestens einer UV-lichtabsorbierenden Gruppe funktionalisiertes Organopolysiloxan, wobei das Verfahren den Schritt des Zugebens zu der topischen Zusammensetzung von hochporöser Kieselsäure und/oder hochporösen vernetzten Polymethylmethacrylatperlen mit einer Teilchengröße Dᵥ0 von größer als 0,3 µm, eine Dᵥ100 von kleiner als 35 µm, eine Dᵥ50, ausgewählt im Bereich von 9 bis 15 µm und einer Ölabsorptionskapazität, ausgewählt im Bereich von 1,2-2,5 cc/g, umfasst.

## Revendications

1. Composition topique comprenant un filtre anti-UV B de type benzimidazole hydrophile, un organopolysiloxane fonctionnalisé par au moins un groupement absorbant la lumière UV, et des billes de silice hautement poreuses et/ou de polyméthacrylate de méthyle réticulé hautement poreuses ayant une taille de particules Dᵥ0 supérieure à 0,3 µm, une Dᵥ100 inférieure à 35 µm, une Dᵥ50 choisie dans la plage allant de 9 à 15 µm, et une capacité d'absorption d'huile choisie dans la plage de 1,2-2,5 cc/g.

2. Composition topique selon la revendication 1, **caractérisée en ce que** la quantité de billes est choisie indépendamment les unes des autres dans la plage de 0,1-5% en poids, sur la base du poids total de la composition cosmétique.

3. Composition topique selon la revendication 1 ou 2, **caractérisée en ce que** la composition ne comprend que des billes de silice.

4. Composition topique selon la revendication 3, **caractérisée en ce que** la capacité d'absorption d'huile des billes de silice est choisie dans la plage allant de 1,3 à 1,8 cc/g.

5. Composition topique selon l'une quelconque des revendications 1 à 4, **caractérisée en ce que** les billes de silice sont en outre revêtues de diméthicone.

6. Composition topique selon la revendication 1 ou 2, **caractérisée en ce que** la composition ne comprend que des billes de polyméthacrylate de méthyle réticulé.

7. Composition topique selon la revendication 6, **caractérisée en ce que** la capacité d'absorption d'huile des billes de polyméthacrylate de méthyle réticulé est choisie dans la plage allant de 1,5 à 2,0 cc/g.

8. Composition topique selon l'une quelconque des revendications 1 à 7, **caractérisée en ce que** le filtre anti-UV B de type benzimidazole est constitué d'acide 2-phénylbenzimidazolsulfonique et l'organopolysiloxane fonctionnalisé par au moins un groupement absorbant la lumière UV est le polysilicone-15.

9. Composition topique selon l'une quelconque des revendications 1 à 8, **caractérisée en ce que** la quantité de filtre anti-UV B de type benzimidazole hydrophile est choisie dans la plage allant de 0,1 à 5% en poids, sur la base du poids total de la composition.

10. Composition topique selon l'une quelconque des revendications 1 à 9, **caractérisée en ce que** la quantité d'organopolysiloxane fonctionnalisé par au moins un groupement absorbant la lumière UV est choisie dans la plage allant de 0,1 à 15% en poids, sur la base du poids total de la composition.

11. Composition topique selon l'une quelconque des revendications 1 à 10, **caractérisée en ce que** la composition comprend en outre au moins un filtre anti-UV supplémentaire choisi dans le groupe constitué par le butylméthoxydibenzoylméthane, l'octocrylène, l'homosalate ou le dioxyde de titane, ainsi que des mélanges de ceux-ci.

12. Composition topique selon l'une quelconque des revendications 1 à 11, **caractérisée en ce que** la composition topique se trouve sous la forme d'une émulsion huile-dans-eau (H/E) comprenant une phase huileuse dispersée dans une phase aqueuse en présence d'un émulsifiant H/E.

13. Composition topique selon la revendication 12, **caractérisée en ce que** l'émulsifiant H/E est le cétylphosphate de potassium.

14. Utilisation de billes de silice hautement poreuses et/ou de polyméthacrylate de méthyle réticulé hautement poreuses ayant une taille de particules Dᵥ0 supérieure à 0,3 µm, une Dᵥ100 inférieure à 35 µm, une Dᵥ50 choisie dans la plage allant de 9 à 15 µm, et une capacité d'absorption d'huile choisie dans la plage de 1,2-2,5 cc/g, afin d'améliorer le FPS dans une composition topique comprenant un filtre anti-UV B de type benzimidazole hydrophile et un organopolysiloxane fonctionnalisé par au moins un groupement absorbant la lumière UV.

15. Méthode d'amélioration du FPS *in vivo* d'une composition topique comprenant un filtre anti-UV B de type benzimidazole hydrophile et un organopolysiloxane fonctionnalisé par au moins un groupement absorbant la lumière UV, ladite méthode comprenant l'étape consistant à ajouter à la composition topique des billes de silice hautement poreuses et/ou de polyméthacrylate de méthyle réticulé hautement poreuses ayant une taille de particules Dᵥ0 supérieure à 0,3 µm, une Dᵥ100 inférieure à 35 µm, une Dᵥ50 choisie dans la plage allant de 9 à 15 µm, et une capacité d'absorption d'huile choisie dans la plage de 1,2-2,5 cc/g.
